(19) Europäisches Patentamt
European Patent Office
Office européen des brevets

(11) Veröffentlichungsnummer: **0 065 658**
**A2**

(12) # EUROPÄISCHE PATENTANMELDUNG

(21) Anmeldenummer: 82103517.7

(22) Anmeldetag: 26.04.82

(51) Int. Cl.[3]: C 07 D 239/42
C 07 C 145/00, C 07 C 145/04
C 07 D 249/14, C 07 D 285/12
C 07 D 263/26, C 07 D 263/58
C 07 D 263/18, C 07 D 265/18
C 07 D 295/22, C 07 D 279/06

(30) Priorität: 07.05.81 DE 3118126

(43) Veröffentlichungstag der Anmeldung:
01.12.82 Patentblatt 82/48

(84) Benannte Vertragsstaaten:
AT BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Busse, Wolf-Dieter, Dr.
Pahlkestrasse 65
D-5600 Wuppertal 1(DE)

(72) Erfinder: Krauthausen, Edmund, Dr.
Franz-Grillparzer-Ring 4
D-5000 Köln 71(DE)

(72) Erfinder: Mardin, Mithat, Dr.
Bergerheide 39
D-5600 Wuppertal 1(DE)

(54) **Verwendung von Sulfenamiden als Lipoxygenasehemmer und diese enthaltende Arzneimittel.**

(57) Die vorliegende Erfindung betrifft die Verwendung von teilweise bekannten Sulfenamiden der allgemeinen Formel I

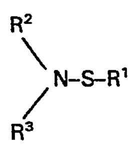

(I)

worin $R^1$, $R^2$ und $R^3$ die in der Beschreibung angegebene Bedeutung haben, als Arzneimittel, insbesondere ihre Verwendung als Lipoxygenasehemmer, diese enthaltende Arzneimittel und deren Herstellung.

EP 0 065 658 A2

Croydon Printing Company Ltd.

BAYER AKTIENGESELLSCHAFT 5090 Leverkusen, Bayerwerk
Zentralbereich KS/bc/c
Patente, Marken und Lizenzen II (Pha)

# Verwendung von Sulfenamiden als Lipoxygenasehemmer und diese enthaltende Arzneimittel

Die vorliegende Erfindung betrifft die Verwendung von teilweise bekannten Sulfenamiden als Arzneimittel, insbesondere ihre Verwendung als Lipoxygenasehemmer, diese enthaltende Arzneimittel und deren Herstellung.

Die erfindungsgemäß verwendeten Sulfenamide sind teilweise bereits bekannt als Anvulkanisationsverzögerer auf dem Kautschukgebiet (vgl. US-Patente 3 546 185, 3 562 225, 3 586 696, 3 645 987, 3 689 467, 3 725 361, 3 862 051, 3 872 061, 3 915 940, 3 993 633, 4 156 680, 4 207 216, Britisches Patent 1 345 144 und die Deutschen Offenlegungsschriften (DOS) 1 620 822, 1 913 725, 2 117 615, 2 136 066, 2 136 090, 2 142 648, 2 164 810, 2 305 555, 2 314 838, 2 337 642 und 2 339 986).

Es ist ebenfalls bekannt, daß die durch das Enzym Lipoxygenase gebildeten Metaboliten der Arachidonsäure an der Entstehung von entzündlichen und allergischen Prozessen beteiligt sind (vgl. E.J. Goetzl,

Immunology 40, 709-719 (1980); Ford-Hutchinson et al. J.Pharm.Pharmacol. 32,517 (1980) und Nature 286, 264 (1980) und Samuelsson, Trends in Pharmacol. Sci., Mai 1980, 227, Borgeat etal., J. Med. Chem. 24,121(1981)).

Bekannte Lipoxygenasehemmer wie Nordihydroguaretsäure, 3-Amino-1-(3-trifluormethylphenyl)-pyrazolin, Phenidon und 5,8,11,14-Eikosatetrainoicsäure sind entweder gleichzeitig als Cyclooxygenasehemmer oder erst bei sehr hohen Konzentrationen wirksam. Die Hemmung des Enzyms Cyclooxygenase des Arachidonsäuremetabolismus führt zu einer globalen Prostaglandinsynthesehemmung und zu einer Stimulation des Lipoxygenaseweges, was eine Gastrotoxizität bzw. proinflammatorische und asthmatische Wirkungen verursacht (vgl. S.S. Yen und W. Kreutner, Agents and Actions, 10, 274 (1980) und G.J. Blackwell und R.J. Flower, Prostaglandins 16, 417 (1978); und vgl. ebenso Brune et al., J.Pharm.Pharmacol. 33, 127-128 (1981); Higgs et al., Eur. J. Pharmacol. 66, 81-86 (1980) und Piper et al, Prostaglandins 19, 371 (1980)). Es besteht ein dringendes Bedürfnis nach Verbindungen, die diese unerwünschten Nebenwirkungen nicht besitzen.

Überraschenderweise hemmen die erfindungsgemäß verwendbaren Sulfenamide die Lipoxygenase sehr spezifisch bereits in solchen Konzentrationen, bei denen die Cyclooxygenase nicht beeinflußt wird. Bei Kenntnis des Standes der Technik konnte diese sehr starke und spezifische Wirkung der Sulfenamide nicht erwartet werden. Die erfindungsgemäßen, Lipoxygenasehemmenden Sulfenamide sind somit als Arzneimittel bei der Behandlung von entzündlichen und allergischen Prozessen einsetzbar. Sie können insbesondere als Antiphlogistika, Antirheumatika, Antiatherosklerotika, Antiasthmatika, Antiallergika, Antimetastatika und Gastroprotektiva Verwendung finden.

Die vorliegende Erfindung betrifft die Verwendung von Sulfenamiden der allgemeinen Formel

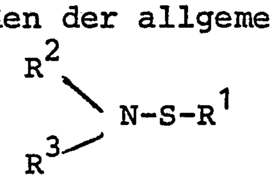

(I),

worin

$R^1$ für Alkyl mit 1 bis 18 C-Atomen, Alkenyl oder Alkinyl mit 2 bis 12 C-Atomen, Aralkyl mit 7 bis 11 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkoxy, Alkyl, Aralkyl, Cycloalkyl, Aryl, Aryloxy, Arylthio, Alkylthio, Carboxy, Carbalkoxy, Cyano, Carbamoyl, Sulfonyl, Halogenalkyl, Halogenalkoxy, Halogen, Amino ode substituiertes Amino substituiert sind, oder $R^1$ für einen der unter $R^2$ genannten Reste steht, wobei $R^2$ die unten angegebene Bedeutung hat, oder $R^1$ für den Rest-$Q-S-NR^2R^3$ steht, wobei $R^2$ und $R^3$ die unten angegebene Bedeutung haben und

Q für Alkylen mit 1 bis 12 C-Atomen, wobei der Alkylrest durch Sauerstoff, Schwefel oder Stickstoff ein- oder mehrfach unterbrochen sein kann, Cycloalkylen mit 5 bis 12 C-Atomen, Arylen mit 6 bis 10 C-Atomen, Alkylen-Cycloalkylen-Alkylen oder Alkylen-Arylen-Alkylen oder einen bivalenten Heterocyclus steht,

$R^2$ einen elektronenziehenden Rest bedeutet, vorzugsweise aus der Gruppe

a) $-CO-R^4$, worin $R^4$ für Wasserstoff, Alkyl, Cycloalkyl, Alkenyl, Aralkyl, Aryl, Alkoxy, Aralkoxy, Aryloxy, Alkylthio, Aralkylthio, Arylthio oder $-NR^3R^5$, worin $R^3$ die weiter unten angegebene Bedeutung hat und $R^5$ Wasserstoff oder einen der unter $R^1$ genannten Reste bedeutet, oder den Rest $-W-CO-NR^3R^5$ bedeutet, wobei W für eine direkte Bindung, eine Disulfidbrücke oder für eines der oben unter Q genannten Brückenglieder steht,

b) heterocyclischer Rest mit 5 bis 8 Ringgliedern, welcher 1 bis 4 Heteroatome aus der Gruppe Sauerstoff, Schwefel oder Stickstoff enthält, wobei dieser heterocyclische Rest gegebenenfalls mit einem Arylrest anelliert ist und bis zu 5 gleiche oder verschiedene Substituenten aus der Gruppe Halogen, Alkylthio, Cycloalkylthio, Aralkylthio, Arylthio, Alkoxy, Aryloxy, Cyano, Nitro, Cycloalkoxy, Aralkoxy, $-NR^3R^5$, $-CO-R^4$, $-SO_2R^6$, $-SR^1$, Trifluormethyl, Trifluormethoxy, Oxo, Thiono, Imino oder substituiertes Imino enthalten kann, wobei als Substituenten der Iminogruppe die oben unter $R^1$ genannten Reste oder ein Carbamoylrest, der am Stickstoff einen Substituenten aus der Gruppe Alkyl, Aryl oder Cycloalkyl trägt, genannt seien,

c) $SO_2-R^6$, worin $R^6$ für Fluoralkyl, einen der unter $R^4$ aufgeführten Reste oder für $-W-SO_2-NR^3R^5$ steht, wobei $R^3$, $R^5$ und W die oben bzw. unten angegebene Bedeutung haben,

d) 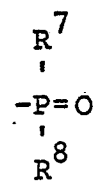

worin $R^7$ und $R^8$ gleich oder verschieden sind und jeweils für eine Gruppe der Formel $-NR^5-SR^1$ oder Wasserstoff oder Halogen oder gegebenenfalls substituiertes Alkyl, Alkenyl, Cycloalkenyl, Aryl, Alkoxy, Alkenoxy, Cycloalkoxy, Aryloxy, Alkylthio, Alkenylthio, Cycloalkylthio, Arylthio, Heterocyclylthio oder Amino stehen oder $R^7$ und $R^8$ gemeinsam mit dem Phosphoratom einen heterocyclischen Ring bilden, und

$R^3$ für einen der unter $R^1$ oder $R^2$ aufgezählten Reste steht oder Wasserstoff, $-S-R^1$ oder $-NR^2R^5$ bedeutet, wobei $R^1$, $R^2$ und $R^5$ die oben angegebene Bedeutung besitzen,

oder

$R^3$ zusammen mit $R^2$ einen gegebenenfalls benz- oder heterocyclisch anellierten heterocyclischen Ring mit 5 bis 12 Ringgliedern bildet, der 1 bis 4 N-Atome, 1 oder 2 O-Atome, 1 oder 2 S-Atome enthalten kann und der durch Alkyl, Cycloalkyl, Alkenyl, Aralkyl, Aryl, Alkoxy, Aryloxy, Alkylmercapto, Mercapto, Amino, Cyano, Halogen, Carbalkoxy, 1 bis 3 Oxo-, 1 oder 2 Thiono- und/oder 1 oder 2

gegebenenfalls substituierte Iminogruppen substituiert sein kann,

sowie ihrer pharmazeutisch unbedenklichen Additionssalze als Arzneimittel.

Für diese Verwendung sind von besonderem Interesse Sulfenamide der allgemeinen Formel (I), in welcher

$R^1$ für ebenfalls substituiertes Alkyl mit 1 bis 18 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl mit 5 bis 7 Kohlenstoffatomen, Phenyl, Phenoxy, Phenylthio, Alkylthio mit 1 bis 4 Kohlenstoffatomen, Carboxy, Carbalkoxy mit 1 bis 4 Kohlenstoffatomen, Cyano, Trifluormethyl, Trifluormethoxy, Fluor, Chlor, Brom, Amino, Alkyl- oder Benzyl-substituiertes Amino, wobei die Alkylreste 1 bis 4 Kohlenstoffatome tragen, substituiert sind,

$R^2$ einen elektronenziehenden Rest bedeutet aus der Gruppe

a) $-CO-R^4$, worin $R^4$ für Wasserstoff, Alkyl mit 1 bis 17 C-Atomen, Cycloalkyl mit 5 bis 8 C-Atomen, Aralkyl mit 7 bis 10 C-Atomen, Aryl mit 6 oder 10 C-Atomen, Alkoxy mit

1 bis 12 C-Atomen, Aralkoxy mit 7 bis 10 C-Atomen, Aryloxy mit 6 oder 10 C-Atomen, Alkylthio mit 1 bis 12 C-Atomen, Benzylthio, Arylthio mit 6 oder 10 C-Atomen oder $-NR^3R^5$ steht, worin $R^3$ die weiter unten angegebene Bedeutung hat und $R^5$ Wasserstoff oder einen der unter $R^1$ genannten Reste bedeutet, oder den Rest $-W-CO-NR^3R^5$ bedeutet, wobei W für eine direkte Bindung oder eine Disulfidbrücke steht,

b) heterocyclischer Rest mit 5 bis 8 Ringgliedern, welcher 1 bis 4 Heteroatome aus der Gruppe Sauerstoff, Schwefel oder Stickstoff enthält, wobei dieser heterocyclische Rest gegebenenfalls mit einem Arylrest mit 6 bis 14 C-Atomen anelliert ist und bis zu 5 gleiche oder verschiedene Substituenten aus der Gruppe Fluor, Chlor, Brom, Alkylthio mit 1 bis 4 C-Atomen, Benzylthio, Phenylthio, Alkoxy mit 1 bis 4 C-Atomen, Phenoxy, Benzyloxy, einen der unter $R^1$ genannten Reste, $-NR^3R^5$, $-CO-R^4$, Trifluormethyl, Trifluormethoxy, Oxo oder Thiono enthalten kann und

$R^3$ für einen der unter $R^1$ oder $R^2$ aufgezählten Reste steht oder Wasserstoff, $-S-R^1$ oder $-NHR^2$ bedeutet, wobei $R^1$ und $R^2$ die oben angegebene Bedeutung besitzen, oder

$R^3$ zusammen mit $R^2$ einen gegebenenfalls benzanellierten heterocyclischen Ring mit 5 bis 12 Ringgliedern bildet, der 1 bis 4 N-Atome und gegebenenfalls 1 oder 2 O-Atome und 1 oder 2 S-Atome enthält und der gegebenenfalls durch Alkyl mit 1 bis 4 C-Atomen, Benzyl, Aryl mit 6 oder 10 C-Atomen, Alkoxy mit 1 bis 4 C-Atomen, Phenoxy, Alkylmercapto mit 1 bis 4 C-Atomen, Amino, Fluor, Chlor, Brom, Carbalkoxy mit 1 bis 4 C-Atomen, 1 bis 3 Oxo-, 1 oder 2 Thiono- und/oder 1 oder 2 Iminogruppen substituiert ist,

sowie ihre pharmazeutisch unbedenklischen Additionssalze.

Die erfindungsgemäß verwendbaren Verbindungen der allgemeinen Formel (I) sind bekannt bzw. lassen sich nach literaturbekannten Methoden, z.B. durch Umsetzung von Sulfenhalogeniden der Formel $R^1$-S-X (II) mit Aminen der Formel Y-$NR^2R^3$ (III) unter Abspaltung von X-Y herstellen (vgl. E. Kühle, The Chemistry of the Sulphenic Acids, Georg Thieme Verlag, Stuttgart 1973, S. 83-91).

Der Rest X steht für Chlor, Brom oder Jod, vorzugsweise für Chlor. Der Rest Y, welcher bei der Reaktion des Amins abgespalten wird, steht vorzugsweise für Wasserstoff, Trialkylsilyl, ein Metall wie K, Na, Li, Mg, Ca, Ba, Ag, Cu, Zn, Fe, Mn, Pb, Sn oder Al oder einen Ammonium-rest.

Für den Fall, daß Y Wasserstoff bedeutet, ist es zweckmäßig, die Umsetzung in Gegenwart einer Base durchzuführen. Als Base seien vorzugsweise genannt

organische Verbindungen wie Triethylamin, Tributylamin, Benzyldimethylamin, Dimethylanilin, Pyridin oder Chinolin. Die Reaktion wird vorzugsweise in Gegenwart eines aprotischen Lösungsmittels wie z.B. Hexan, Ligroin, Benzol, Toluol, Chlorbenzol, Chloroform, Tetrachlorkohlenstoff, Dimethylsulfoxid oder Dimethylformamid durchgeführt.

Für den Fall, daß Y nicht für einen Trialkylsilylrest steht, kann es vorteilhaft sein, die Herstellung in einem wäßrig-organischen Zweiphasenmedium durchzuführen. In diesem Falle können auch anorganische Basen als Halogenwasserstoff-Fänger eingesetzt werden, wie z.B. Alkali- oder Erdalkalihydroxide oder -carbonate.

Die Reaktion kann bei Temperaturen zwischen -80 und +150°C, vorzugsweise zwischen 0 und 50°C, durchgeführt werden.

Die für die Durchführung der Erfindung geeigneten Amine der allgemeinen Formel (III) sind bekannt oder können nach bekannten Methoden hergestellt werden (Literatur: Houben-Weyl, Methoden der Organischen Chemie, Bd. XI/2, 4. Aufl., G. Thieme Verlag, Stuttgart 1958, S. 3 ff; Aminoheterocyclen: s. A. Weissberger, The Chemistry of Heterocyclic Compounds, Interscience, 1950-79; S. Coffey, Rodd's Chemistry of Carbon Compounds, 2. Aufl. Vol. IV A-K, Elsevier Publ., Amsterdam-New York-London 1973-79).

Die für die Durchführung der Erfindung geeigneten Sulfenhalogenide der allgemeinen Formel (II) sind bekannt oder können ebenfalls nach bekannten Methoden hergestellt werden (vgl. E. Kühle, The Chemistry of the Sulphenic Acids, G. Thieme Verlag, Stuttgart 1973, S. 2-37).

Der Nachweis der lipoxygenasehemmenden Eigenschaften der Sulfenamide erfolgt analog der Methode von Bailey et al., J. Biol. Chem. 255, 5996, (1980) und nach R.H. Flower, Prostaglandins 16, 417 (1978). Bei dieser Testmethode wird der Metabolismus radioaktiv markierter Arachidonsäure an gewaschenen Humanthrombozyten untersucht. Bei diesem in vitro Test werden die radioaktiv markierten Metaboliten aus dem Reaktionsansatz extrahiert und dünnschichtchromatographisch getrennt. Das Autoradiogramm wird am Dünnschicht-Scanner ausgewertet. Bei diesen Testbedingungen werden die markierten Metaboliten von der nicht umgesetzten Arachidonsäure getrennt und sind anschließend quantitativ auswertbar. Die Verteilung der Radioaktivität auf die während der Metabolisierung gebildeten Cyclooxygenaseprodukte Thromboxan $B_2$ ($TXB_2$) und 12-Hydroxy-5,8,10-heptadekatriensäure (HHT) bzw. das Lipoxygenaseprodukt 12-Hydroxy-5,8,11,14-eikosatetraensäure (HETE) unter dem Einfluß der Inhibitoren stellt ein Maß für die Hemmung der Enzyme dar.

Die Lipoxygenasehemmung der erfindungsgemäß verwendbaren Sulfenamide läßt sich an der Hemmung der HETE-Synthese messen. Es zeigt sich, daß die Synthese von $TXB_2$ und von HHT unbeeinflußt bleibt, während der Arachidonsäureumsatz abnimmt. Wie aus der nachfolgenden Tabelle ersichtlich ist, bewirken die Sulfenamide eine signifikante Hemmung der Plättchenlipoxygenase (HETE-Synthese).

Hemmung der Plättchenlipoxygenase (HETE-Synthese)

| Verbindung aus Beispiel Nr. | Minimale effektive Hemmkonzentration (g/ml) (mindestens 50 % Hemmung) |
|---|---|
| 2 | $10^{-6}$ |
| 3 | $10^{-6}$ |
| 17 | $3 \times 10^{-6}$ |
| 18 | $3 \times 10^{-6}$ |
| 19 | $10^{-6}$ |
| 23 | $10^{-6}$ |
| 24 | $10^{-6}$ |
| 25 | $10^{-6}$ |
| 29 | $10^{-6}$ |
| 30 | $3 \times 10^{-6}$ |
| 31 | $3 \times 10^{-6}$ |
| 32 | $3 \times 10^{-6}$ |
| 35 | $3 \times 10^{-6}$ |
| 39 | $10^{-6}$ |
| 40 | $3 \times 10^{-6}$ |
| 41 | $10^{-6}$ |
| 42 | $3 \times 10^{-6}$ |
| 43 | $3 \times 10^{-6}$ |
| 52 | $10^{-6}$ |
| 53 | $10^{-6}$ |

Hemmung der Plättchenlipoxygenase (HETE-Synthese)

(Fortsetzung)

| Verbindung aus Beispiel Nr. | Minimale effektive Hemmkonzentration (g/ml) (mindestens 50 % Hemmung) |
|---|---|
| 54 | $10^{-6}$ |
| 55 | $10^{-6}$ |
| 56 | $3 \times 10^{-6}$ |
| 57 | $10^{-5}$ |
| 60 | $3 \times 10^{-6}$ |
| 61 | $10^{-6}$ |
| 62 | $3 \times 10^{-6}$ |
| 68 | $3 \times 10^{-6}$ |
| 71 | $3 \times 10^{-6}$ |
| 72 | $10^{-6}$ |
| 73 | $10^{-6}$ |
| 74 | $10^{-6}$ |
| 75 | $3 \times 10^{-6}$ |
| 76 | $3 \times 10^{-6}$ |
| 80 | $10^{5}$ |
| 84 | $10^{5}$ |

Die erfindungsgemäßen Sulfenamide sind auch in vivo wirksam. Diese Wirkung wird durch die Messung der Hemmung der Leukozytenmigration nach an sich bekannten Methoden nachgewiesen (vgl. Higgs et al., Biochemical Pharmacology 28, 1959, (1979) und Eur. J. Pharmacol. 66, 81 (1980)). In der nachfolgenden Tabelle 2 sind die Wirkungen einiger beispielhafter Sulfenamide nach lokaler Applikation, durch Einführen eines mit Wirkstoff getränkten Schwämmchens unter die Rückenhaut von Ratten, zusammengefaßt.

Tabelle 2

| Verbindung Nr. | Dosis, lokal (mg/Ratte) | Hemmung der Leukozytenmigration (Kontrolle = 0 %) |
|---|---|---|
| 2 | 10 | 70 % |
| 4 | 10 | 61 % |
| 5 | 10 | 22 % |
| 10 | 10 | 26 % |
| 11 | 10 | 22 % |
| 15 | 10 | 47 % |
| 16 | 10 | 40 % |
| 18 | 10 | 41 % |
| 19 | 10 | 60 % |
| 20 | 10 | 64 % |
| 21 | 10 | 44 % |
| 22 | 10 | 64 % |
| 29 | 10 | 48 % |

Tabelle 2 (Fortsetzung)

| Verbindung Nr. | Dosis, lokal (mg/Ratte) | Hemmung der Leukozytenmigration (Kontrolle = 0 %) |
|---|---|---|
| 31 | 10 | 43 % |
| 32 | 10 | 38 % |
| 36 | 10 | 64 % |
| 38 | 10 | 56 % |
| 40 | 10 | 41 % |
| 45 | 10 | 40 % |
| 47 | 10 | 21 % |
| 52 | 10 | 76 % |
| 55 | 10 | 78 % |
| 56 | 10 | 86 % |
| 58 | 10 | 78 % |
| 69 | 10 | 20 % |
| 71 | 10 | 65 % |
| 74 | 10 | 52 % |

Die antiasthematische Wirkung der erfindungsgemäßen Verbindung kann ebenfalls nach bereits bekannten Methoden nachgewiesen werden (vgl. Samuelson et al., FEBS Letters, 110, 213 (1980) und Yen et al., Agents and Actions 10, 274 (1980)).

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nicht-ionogene

anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspension und/oder Elixieren, die für orale Anwendungen gedacht sind, können die Wirkstoffe außer mit den o.g. Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse

zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Die folgenden Beispiele sollen die Erfindung näher erläutern:

Beispiel 1

Zur Suspension von 47,5 g (0,5 Mol) 2-Pyrimidinamin und 44 g Pyridin in 100 ml trockenem Dimethylformamid tropft man eine Lösung von 0,5 Mol Trichloromethyl in 300 ml Methylchlorid. Man läßt 2 Stunden bei Raumtemperatur nachrühren, saugt vom ausgefallenen Pyridinhydrochlorid ab und schüttelt die Mutterlauge zweimal mit Eiswasser aus. Die organische Phase wird über Natriumsulfat getrocknet, das Lösungsmittel im Vakuum abgedampft und der Rückstand aus Toluol umkristallisiert. Man erhält 30 g N-2-Pyrimidin-trichlormethylsulfenamid als weiße Kristalle vom Schmp. 178-179°C.

Beispiele 2-78

Analog Beispiel 1 wurden die folgenden Beispiele erhalten, s. Tabelle 3. In einigen Fällen waren die erhaltenen Sulfenamide schwer löslich, so daß sie durch Abfiltrieren und Waschen mit Eiswasser isoliert werden konnten.

Tabelle 3

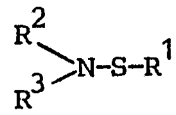

| Bei-spiel | $-NR^2R^3$ | $-R^1$ | Verwendetes Lösungsmittel | Ausbeute (%) | Schmp. (°C) (Umkrist. aus) 1) | Bemerkungen |
|---|---|---|---|---|---|---|
| 2 | 3-Amino-1,2,4-triazolyl | Phenyl | Toluene/DMF | 61 | 154°C (EE) | |
| 3 | " | 4-Chlorphenyl | " " | 46 | 154-6°C (EE) | Bildet sich beim Umkrist. aus Bsp. 10 |
| 4 | " | 4-Methylphenyl | " " | 43 | 130-2°C | |
| 5 | 5-(Morpholin-4-yl)-1,3,4-thiadiazol-2-yl-NH | 4-Chlorphenyl | " / " | 94 | 143-5 | |
| 6 | 5-Phenoxymethyl-oxazolidin-2-on-3-yl | " | " / " | 33 | 98-101 | |
| 7 | 3a,4,5,6,7,7a-Hexahydrobenz-oxazolin-2-on-3-yl | " | " / " | 92 | zähes, gelbes Öl | |
| 8 | 3-Phenyl-1,2,4-thiadiazol-5-yl-NH | 1-Phenyl-tetrazol-5-yl | Chlorbenzol/Dimethylacetamid | 97 | 154 | |

Tabelle 3 (Fortsetzung)

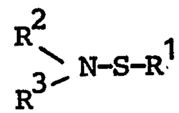

| Beispiel | $-NR^2R^3$ | $-R^1$ | Verwendetes Lösungsmittel | Ausbeute (%) | Schmp. (°C) (Umkrist. aus) 1) | Bemerkungen |
|---|---|---|---|---|---|---|
| 9 | 4-Phenylthiazol-2-yl-NH | 1-Phenyl-tetrazol-5-yl | Chlorbenzol/ Dimethylacetamid | 40 | 152 | |
| 10 | Benzyl-NH- | " | " / " | 62 | 46 | |
| 11 | 1,2,4-Triazol-3-yl-NH | " | " / " | 35 | 175 | |
| 12 | " | Benzothiazol-2-yl | " / " | 15 | 177° | |
| 13 | N-Acetyl-4-Ethoxyanilin | 4-Chlorphenyl | Toluol/DMF | 73 | gelbes, klares Öl | |
| 14 | N-Acetyl-4- | " | " / " | 68 | h'braunes Öl | |
| 15 | 2,2,5,5-Tetramethyloxazolidin-4-on-3-yl | " | " / " | 53 | 61-4°C | |
| 16 | " | 4-Methylphenyl | " / " | 74 | braunes Öl | Krist.nach längerem Stehen durch |

Tabelle 3 (Fortsetzung)

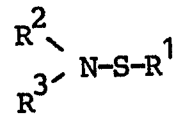

| Bei-spiel | $-NR^2R^3$ | $-R^1$ | Verwendetes Lösungsmittel | Ausbeute (%) | Schmp. (°C) (Umkrist. aus) 1) | Bemerkungen |
|---|---|---|---|---|---|---|
| 17 | d-Benzoxazin-2,6-dion-3-yl | 4-Chlor-phenyl | Toluol/DMF | 81 | 190 (Zers.) | |
| 18 | Triazo/1,2-a/-triazol-1,3,5,7-tetraon-2,6-diyl | Phenyl | " / " | 45 | 143 (Zers.) (Tol) | Enthält noch etwas Mono-sulfenyliertes Produkt |
| 19 | tert.-Butylcarba-moyl | " | THF | 33 | 103 | säulenchromato-graphisch ge-reinigt |
| 20 | N-Pyrrolidonyl | 4-Chlor-3-Tri-fluor-methyl-phenyl | Chlorbenzol/DMF | 42 | 78-80 | |
| 21 | " | 2-Chlor-5-Tri-fluormethyl-phenyl | Toluol/ " | 45 | 97-100 | |

Tabelle 3 (Fortsetzung)

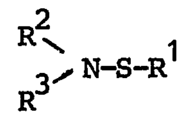

| Beispiel | $-NR^2R^3$ | $-R^1$ | Verwendetes Lösungsmittel | Ausbeute (%) | Schmp. (°C) (Umkrist. aus) 1) | Bemerkungen |
|---|---|---|---|---|---|---|
| 22 | N-Pyrrolidonyl | 4-Carbomethoxyphenyl | Toluol/DMF | 50 | 76-8 | Kristallisierte nach Verreiben mit Cyclohexan |
| 23 | N-Phenylthio-2-Chlorphenylcarbamoyl | Phenyl | THF | 32 | 113-5 (Ethanol) | |
| 24 | 2-Chlorphenylcarbamoyl | Phenyl | THF | 3 | 116-8 (Tol) | Aus der Mutterlauge von Beispiel 104 isoliert |
| 25 | Chlormethylcarbamoyl | Phenyl | THF | 29 | 95-7 (Tol) | |
| 26 | 6-Carbomethoxy-1,3-thiazin-4-on-2-yl-NH | 4-Chlorphenyl | Toluol/DMF | 98 | 165-8 | |
| 27 | 2-Benzothiazolyl | Phenyl | " " | 19 | 150-4 (EE) | |
| 28 | 6-Methoxy-2-benzothiazolyl | Phenyl | " " | 82 | 165-6 (EE) | |
| 29 | Phthalimidyl | Phenyl | " " | 92 | 153-8 | |

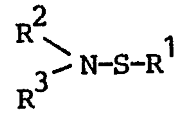

Tabelle 3 (Fortsetzung)

| Bei-spiel | $-NR^2R^3$ | $-R^1$ | Verwendetes Lösungsmittel | Ausbeute (%) | Schmp. (°C) (Umkrist. aus) 1) | Bemerkungen |
|---|---|---|---|---|---|---|
| 30 | " | 4-Chlorphenyl | Toluol/DMF | 77 | 163-5 | |
| 31 | " | 4-Methylphenyl | " " | 77 | 190-2 | |
| 32 | " | Cyclohexyl | Toluol/Aceton | 77 | 85-7 (WB) | |
| 33 | " | Isopropyl | Hexan/DMF | 32 | 63-5 (WB) | |
| 34 | " | 2-Carbomethoxy-phenyl | Toluol/DMF | 77 | 244-6 | |
| 35 | " | 4-tert.-Butyl-phenyl | " " | 55 | 155-7 | |
| 36 | 5-Nitrophthal-imidyl | Phenyl | Toluol/DMF | 81 | 136-8 | |
| 37 | " | 4-tert.-Butylphenyl | " " | 40 | 146-8 | |
| 38 | " | 4-Chlorphenyl | Chlorbenzol/DMF | 44 | 181-3 (EE) | |
| 39 | 4-Nitrophthal-imidyl | Phenyl | Toluol/DMF | 80 | 149-54 | |
| 40 | " | 4-Chlorphenyl | Chlorbenzol/DMF | 71 | 160-7 | |
| 41 | " | 4-tert.-Butylphenyl | Toluol/DMF | 61 | 136-7 | |

Tabelle 3 (Fortsetzung) 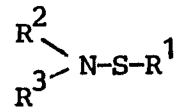

| Beispiel | $-NR^2R^3$ | $-R^1$ | Verwendetes Lösungsmittel | Ausbeute (%) | Schmp. (°C) (Umkrist. aus) 1) | Bemerkungen |
|---|---|---|---|---|---|---|
| 42 | Tetrachlorphthalimidyl | Phenyl | Toluol/DMF | 65 | 207 | |
| 43 | " | 4-tert.-Butylphenyl | " " | 54 | | |
| 44 | " | 4-Chlorphenyl | " " | 24 | 227-31 (Tol) | |
| 45 | 1,2,4,5-Benzol-tetracarbon-säurediimid-N,N'-diyl | Phenyl | " " | 64 | 238 | |
| 46 | 1,2,4,5-Benzol-tetracarbon-säurediimid-N,N'-diyl | 4-Chlorphenyl | Chlorbenzol/DMF | 59 | 238-40 | |
| 47 | N-Naphthalimidyl | Phenyl | Toluol/DMF | 94 | 205-12 | |
| 48 | " | 4-Chlorphenyl | Chlorbenzol/DMF | 84 | 191-3 | |
| 49 | " | 4-tert.-Butylphenyl | Toluol/DMF | 69 | 196-8 | |
| 50 | N-Phthalimidyl | Benzoylmethyl | Chlorbenzol | 78 | 144-7 (WB) | |
| 51 | " | 2-Acetyl-2-propyl | " | 86 | 101-6 (Cy) | |
| 52 | N-Pyrrolidonyl | Phenyl | Toluol/DMF | 81 | | |

Tabelle 3 (Fortsetzung)

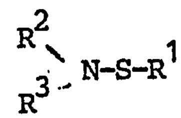

| Beispiel | $-NR^2R^3$ | $-R^1$ | Verwendetes Lösungsmittel | Ausbeute (%) | Schmp. (°C) (Umkrist. aus) 1) | Bemerkungen |
|---|---|---|---|---|---|---|
| 53 | " | 4-Chlorphenyl | Chlorbenzol/DMF | 46 | 78-80 (Cy) | |
| 54 | " | 4-tert.-Butylphenyl | " " | 94 | Öl | |
| 55 | Hexahydroazepin-2-on-1-yl | Phenyl | Toluol/DMF | 38 | 63-8 | |
| 56 | " | 4-Chlorphenyl | " " | 64 | 79-81 (WB) | |
| 57 | " | 4-tert.-Butylphenyl | " " | 58 | 76-80 | |
| 58 | 2-Oxo-Azacyclododecanyl | Phenyl | Toluol/DMF | 59 | Öl | |
| 59 | Phenylcarbamoyl | Phenyl | THF | 23 | 122-4 (Tol) | |
| 60 | N-Saccharinyl | Phenyl | Chlorbenzol | 91 | 139-41 | |
| 61 | " | 4-Chlorphenyl | " | 69 | 202 | |
| 62 | " | 4-tert.-Butylphenyl | " | 87 | 143 | |
| 63 | " | 2-Nitrophenyl | " | 62 | 170 | |
| 64 | " | 2-Formyl-2-propyl | " | 83 | 133-5 | |
| 65 | 5,5-Dimethylhydantoin-1,3-diyl | Phenyl | Toluol/DMF | 26 | 104 | |
| 66 | " | 4-tert.-Butylphenyl | " " | 66 | 140-2 (Cy/Tol) | |

Tabelle 3 (Fortsetzung)

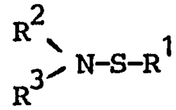

| Beispiel | $-NR^2R^3$ | $-R^1$ | Verwendetes Lösungsmittel | Ausbeute (%) | Schmp. (°C) (Umkrist. aus) 1) | Bemerkungen |
|---|---|---|---|---|---|---|
| 67 | N-Isatinyl | Cyclohexyl | Dichlorethan/DMF | 75 | 101-4 | |
| 68 | " | Phenyl | Chlorbenzol/DMF | 66 | 118-23 | |
| 69 | " | 4-Chlorphenyl | " " | 93 | 180-2 | |
| 70 | " | 4-tert.-Butylphenyl | " " | 63 | 112-5 | |
| 71 | 1-(Phenylcarbonatimino)-isoindolin-3-on-2-yl | Phenyl | Toluol/DMF | 66 | 133-50 | |
| 72 | 1-Phenyl-pyrazolidin-3-on-2-yl | 4-Chlorphenyl | Chlorbenzol/DMF | 14 | 194-7 (Tol) | |
| 73 | " | 4-tert.-Butylphenyl | " " | 12 | 170-2 (Cy/Tol) | |
| 74 | 4,5-Dihydro-3-methyl-pyrazol-5-on-1-yl | Phenyl | Toluol/DMF | 55 | 290 | |
| 75 | 1-Pyrazolyl | 4-Chlorphenyl | " " | 65 | Öl | |
| 76 | e-Benzoxazin-2,4-dion-3-yl | Cyclohexyl | Pentan/DMF | 72 | 105-8 (iPr) | |
| 77 | 3-Methyl-1,2,4-thiadiazol-5-yl-NH | 4-Chlor-3-Trifluormethylphenyl | Toluol/DMF | 30 | 123-7 | Mehrmals mit Petrolether gewaschen |
| 78 | 5-Methyl-1,3,4-thiadiazol-2-yl-NH | 4-Fluorphenyl | " /" | 50 | 132-4 (Cy/Tol) | |

Erläuterungen zu Tabelle 3

1)

Cy = Cyclohexan

EE = Essigsäureethylester

iPr = i-Propanol

Me = Methanol

Tol = Toluol

WB = Waschbenzin (Kp. 100-140°C)

Beispiel 79

42,5 g (0,5 Mol) 5-Aminotetrazol werden in 100 ml Wasser und 20 g (0,5 Mol) Natronlauge gelöst. Man gibt 100 ml Chlorbenzol zu und tropft dann bei +10 °C eine Lösung aus 0,5 Mol Trichloromethylsulfenchlorid in 400 ml Chlorbenzol langsam zu. Nach beendeter Zugabe läßt man 30 Minuten nachrühren und nutscht dann ab. Der Rückstand wird mit Petrolether gewaschen und dann über $P_4O_{10}$ getrocknet. Man erhält 66 % der Theorie an N-5-Tetrazolyl-trichloromethylsulfenamid mit Schmp. 117 to 118 °C. (Zers.).

Beispiel 80

Zu 80,4 g Trimethylchlorsilan in 500 ml trockenem Toluol leitet man bei 50 bis 60 °C bis zur Sättigung $NH_3$-Gas ein. Anschließend trägt man 59,1 g Benzimidazol ein und kocht 10 Stunden unter Rückfluß. Zur Entfernung von überschüssigem Ammoniak destilliert man im Vakuum 250 ml Toluol ab und tropft anschließend 0,5 Mol Cyclohexylsulfenchlorid in 500 ml Pentan zu.

Man rührt 4 Stunden nach, filtriert und entfernt das Lösungsmittel im Vakuum. Man erhält 99 g 1-Cyclohexylthiobenzimidazol als rotbraunes Öl.

Analog Beispiel 80 wurden auch die Beispiele 81 bis 86 erhalten, s. Tabelle 4.

Tabelle 4

$R^2R^3N-S-R^1$

| Beispiel | $-NR^2R^3$ | $-R^1$ | Lösungsmittel | Ausbeute (%) | Schmp. (°C) (Umkrist. aus) | Bemerkungen |
|---|---|---|---|---|---|---|
| 81 | 1-Benzimidazolyl | Phenyl | Dichlorethan/Toluol | 56 | 50-4 | Nach 2 Wochen ausgefallene Kristalle mit Petrolether gewaschen |
| 82 | " | Isopropyl | " " | 65 | Öl | |
| 83 | " | n-Octyl | " " | 92 | Öl | |
| 84 | " | sec.-Butyl | Pentan/Toluol | 49 | $Kp_{0,1}$ 115-120 °C (gelbes Öl) | |
| 85 | 1-Imidazolyl | Cyclohexyl | " " | 72 | gelbes Öl | Nebenkomponente Dicyclohexyldisulfid wurde bei 0,3 mbar und 70 °C Badtemperatur entfernt |
| 86 | " | n-Octyl | " " | 90 | gelbes Öl | |

Patentansprüche

1) Sulfenamide der allgemeinen Formel (I)

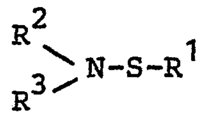 (I)

in welcher

$R^1$ für Alkyl mit 1 bis 18 C-Atomen, Alkenyl oder Alkinyl mit 2 bis 12 C-Atomen, Aralkyl mit 7 bis 11 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkoxy, Alkyl, Aralkyl, Cycloalkyl, Aryl, Aryloxy, Arylthio, Alkylthio, Carboxy, Carbalkoxy, Cyano, Carbamoyl, Sulfonyl, Halogenalkyl, Halogenalkoxy, Halogen, Amino oder substituiertes Amino substituiert sind, oder $R^1$ für einen der unter $R^2$ genannten Reste steht, wobei $R^2$ die unten angegebene Bedeutung hat, oder $R^1$ für den Rest $-Q-S-NR^2R^3$ steht, wobei

$R^2$ und $R^3$ die unten angegebene Bedeutung haben und

Q für Alkylen mit 1 bis 12 C-Atomen, wobei der Alkylenrest durch Sauerstoff, Schwefel oder Stickstoff ein- oder mehrfach unterbrochen sein kann, Cycloalkylen mit 5 bis 12 C-Atomen, Arylen mit 6 bis 10

C-Atomen, Alkylen-Cycloalkylen-Alkylen oder Alkylen-Arylen-Alkylen oder einen bivalenten Heterocyclus steht,

$R^2$ einen elektronenziehenden Rest bedeutet, vorzugsweise aus der Gruppe

a) $-CO-R^4$, worin $R^4$ für Wasserstoff, Alkyl, Cycloalkyl, Alkenyl, Aralkyl, Aryl, Alkoxy, Aralkoxy, Aryloxy, Alkylthio, Aralkylthio, Arylthio oder , $-NR^3R^5$, worin $R^3$ die weiter unten angegebene Bedeutung hat und $R^5$ Wasserstoff oder einen der unter $R^1$ genannten Reste bedeutet, oder den Rest $-W-CO-NR^3R^5$ bedeutet, wobei W für eine direkte Bindung, eine Disulfidbrücke oder für eines der oben unter Q genannten Brückenglieder steht,

b) heterocyclischer Rest mit 5 bis 8 Ringgliedern, welcher 1 bis 4 Heteroatome aus der Gruppe Sauerstoff, Schwefel oder Stickstoff enthält, wobei dieser heterocyclische Rest gegebenenfalls mit einem Arylrest anelliert ist und bis zu 5 gleiche oder verschiedene Substituenten aus der Gruppe Halogen, Alkylthio, Cycloalkylthio, Aralkylthio, Arylthio, Alkoxy, Aryloxy, Cyano, Nitro, Cycloalkoxy, Aralkoxy, $-NR^3R^5$, $-CO-R^4$, $-SO_2R^6$, $-S-R^1$, Trifluormethyl, Trifluormethoxy, Oxo, Thiono, Imino oder substituiertes Imino enthalten kann, wobei als Substituenten der Iminogruppe die oben unter $R^1$ genannten Reste oder ein Carbamoylrest, der am Stickstoff einen Substituenten aus der Gruppe Alkyl, Aryl oder Cycloalkyl trägt, genannt

seien,

c) $SO_2-R^6$, worin $R^6$ für Fluoralkyl, einen der unter $R^4$ aufgeführten Reste oder für $-W-SO_2-NR^3R^5$ steht, wobei $R^3$, $R^5$ und W die oben bzw. unten angegebene Bedeutung haben,

d) 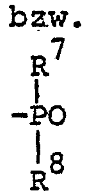

worin $R^7$ und $R^8$ gleich oder verschieden sind und jeweils für eine Gruppe der Formel $-NR^5-SR^1$ oder Wasserstoff oder Halogen oder gegebenenfalls substituiertes Alkyl, Alkenyl, Cycloalkenyl, Aryl, Alkoxy, Alkenoxy, Cycloalkoxy, Aryloxy, Alkylthio, Alkenylthio, Cycloalkylthio, Arylthio, Heterocyclylthio oder Amino stehen oder $R^7$ und $R^8$ gemeinsam mit dem Phosphoratom einen heterocyclischen Ring bilden und

$R^3$ für einen der unter $R^1$ oder $R^2$ aufgezählten Reste steht oder Wasserstoff, $-S-R^1$, oder $-NR^2R^5$ bedeutet, wobei $R^1$, $R^2$ und $R^5$ die oben angegebene Bedeutung besitzen,

oder

$R^3$ zusammen mit $R^2$ einen gegebenenfalls benz- oder heterocyclisch anellierten heterocyclischen Ring mit 5 bis 12 Ringgliedern bildet, der 1 bis 4 N-Atome, 1 oder 2 O-Atome, 1 oder 2 S-Atome enthalten kann und der durch Alkyl, Cycloalkyl, Alkenyl, Aralkyl, Aryl, Alkoxy, Aryloxy, Alkylmercapto, Mercapto,

Amino, Cyano, Halogen, Carbalkoxy, 1 bis 3 Oxo-, 1 oder 2 Thiono- und/oder 1 oder 2 gegebenenfalls substituierte Iminogruppen substituiert sein kann,

sowie ihrer pharmazeutisch unbedenklichen Additionssalze, zur Verwendung in Arzneimitteln.

2) Sulfenamide der allgemeinen Formel (I), in welcher

$R^1$ für Alkyl mit 1 bis 18 C-Atomen, Cycloalkyl mit 5 bis 8 C-Atomen oder für Aryl mit 6 bis 14 C-Atomen steht, wobei diese Reste gegebenenfalls mit bis zu 5 gleichen oder verschiedenen Substituenten aus der Gruppe Alkyl, Alkoxy mit jeweils 1 bis 4 Kohlenstoffatomen, Aralkyl mit 7 bis 10 Kohlenstoffatomen, Cycloalkyl mit 5 bis 7 Kohlenstoffatomen, Phenyl, Phenoxy, Phenylthio, Alkylthio mit 1 bis 4 Kohlenstoffatomen, Carboxy, Carbalkoxy mit 1 bis 4 Kohlenstoffatomen, Cyano, Trifluormethyl, Trifluormethoxy, Fluor, Chlor, Brom, Amino, Alkyl- oder Benzylsubstituiertes Amino, wobei die Alkylreste 1 bis 4 Kohlenstoffatome tragen, substituiert sind,

$R^2$ einen elektronenziehenden Rest bedeutet aus der Gruppe

a) $-CO-R^4$, worin $R^4$ für Wasserstoff, Alkyl mit 1 bis 17 C-Atomen, Cycloalkyl mit 5 bis 8 C-Atomen, Aralkyl mit 7 bis 10 C-Atomen, Aryl mit 6 oder 10 C-Atomen, Alkoxy mit 1 bis 12 C-Atomen, Aralkoxy mit 7 bis 10 C-Atomen, Aryloxy mit 6 oder 10 C-Atomen, Alkylthio mit 1 bis 12 C-Atomen, Benzylthio, Arylthio mit 6 oder 10 C-Atomen, $-NR^3R^5$, worin $R^3$ die weiter unten angegebene Bedeutung hat und $R^5$ Wasserstoff oder einen der unter $R^1$ genannten Reste bedeutet, oder den Rest $-W-CO-NR^3R^5$ bedeutet, wobei W für eine direkte Bindung oder eine Disulfidbrücke steht,

b) heterocyclischer Rest mit 5 bis 8 Ringgliedern, welcher 1 bis 4 Heteroatome aus der Gruppe Sauerstoff, Schwefel oder Stickstoff enthält, wobei dieser heterocyclische Rest gegebenenfalls mit einem Arylrest mit 6 bis 14 C-Atomen anelliert ist und bis zu 5 gleiche oder verschiedene Substituenten aus der Gruppe Fluor, Chlor, Brom, Alkylthio mit 1 bis 4 C-Atomen, Benzylthio, Phenylthio, Alkoxy mit 1 bis 4 C-Atomen, Phenoxy, Benzyloxy, einen der unter $R^1$ genannten Reste, $-NR^3R^5$, $-CO-R^4$, Trifluormethyl, Trifluormethoxy, Oxo oder Thiono enthalten kann und

$R^3$ für einen der unter $R^1$ oder $R^2$ aufgezählten Reste steht oder Wasserstoff, $-S-R^1$ oder $-NHR^2$ bedeutet, wobei $R^1$ und $R^2$ die oben angegebene Bedeutung besitzen, oder

$R^3$ zusammen mit $R^2$ einen gegebenenfalls benzanellierten heterocyclischen Ring mit 5 bis 12 Ringgliedern bildet, der 1 bis 4 N-Atome, 1 oder 2 O-Atome oder 1 oder 2 S-Atome enthält und der gegebenenfalls durch Alkyl mit 1 bis 4 C-Atomen, Benzyl, Aryl mit 6 oder 10 C-Atomen, Alkoxy mit 1 bis 4 C-Atomen, Phenoxy, Alkylmercapto mit 1 bis 4 C-Atomen, Amino, Fluor, Chlor, Brom, Carbalkoxy mit 1 bis 4 C-Atomen, 1 bis 3 Oxo-, 1 oder 2 Thiono- und/ oder 1 oder 2 Iminogruppen substituiert ist,

sowie ihre pharmazeutisch unbedenklichen Additionssalze, zur Verwendung in Arzneimitteln.

3) Verwendung von Sulfenamiden als Arzneimittel.

4) Verwendung von Sulfenamiden der allgemeinen Formel (I) gemäß Anspruch 1 als Arzneimittel.

5) Verwendung von Sulfenamiden gemäß Anspruch 2 als Arzneimittel.

6) Verwendung von Sulfenamiden als Lipoxygenasehemmer.

7) Verwendung von Sulfenamiden gemäß Anspruch 1 als Antiphlogistika, Antirheumatika, Antiatherosklerotika, Antiasthmatika, Antiallergika, Antimetastatika und Gastroprotektiva.

8) Arzneimittel enthaltend mindestens eine Verbindung der allgemeinen Formel (I) gemäß Anspruch 1.

9) Arzneimittel mit Lipoxygenase-hemmender Wirkung, enthaltend mindestens eine Verbindung der allgemeinen Formel (I) gemäß Anspruch 1.

10) Verfahren zur Herstellung von Arzneimitteln, dadurch gekennzeichnet, daß man Sulfenamide der allgemeinen Formel (I) gemäß Anspruch 1, gegebenenfalls zusammen mit Hilfs- und Trägerstoffen in eine geeignete Applikationsform überführt.